# EUROPEAN PATENT SPECIFICATION

(11) **EP 1 404 309 B1**
(45) Date of publication and mention of the grant of the patent: **23.08.2006**
(21) Application number: 02728300.1
(22) Date of filing: 30.05.2002
(51) Int. Cl.: A61K 31/19, A61K 31/325, A61K 31/155, A61P 3/10, A61P 5/50

(54) **A PHARMACEUTICAL COMBINATION COMPRISING EITHER (S)-2-ETHOXY-3- 4-(2- 4-METHANE SULFONYL OXYPHENYL ETHOXY) PHENYL| PROPANOIC ACID OR 3- 4- 2- (4-TERT-BUTOXY CARBONYL AMINOPHENYL) ETHOXY| PHENYL -(S)-2-ETHOXY PROPANOIC ACID AND A BIGUANIDE DRUG**
PHARMAZEUTISCHE KOMBINATION MIT (S)-2-ETHOXY-3-4-(2-4-METHANSULFONYLOXYPHENYLETHOXY) PHENYL PROPANSÄURE ODER 3-4-2-(4-TERT.-BUTOXYCARBONYLAMINOPHENYL) ETHOXYPHENYL-(S)-2-ETHOXYPROPANSÄURE UND EINEM BIGUANID-ARZNEIMITTEL
PREPARATION PHARMACEUTIQUE COMPRENANT SOIT DE L'ACIDE (S)-2-ETHOXY-3- 4-(2- 4-METHANESULFONYLOXYPHENYL ETHOXY)PHENYL| PROPANOIQUE, SOIT DE L'ACIDE 3- 4- 2-(4-$I(TERT)-BUTOXYCARBONYLAMINOPHENYL)ETHOXY|PHENYL -(S)-2-ETHOXY PROPANOIQUE, ET UN MEDICAMENT DE BIGUANIDE.

(30) Priority: 01.06.2001 SE 0101980
(43) Date of publication of application: 07.04.2004
(60) Divisional application: 06000170.8
(73) Proprietor: AstraZeneca AB, 151 85 Södertälje (SE)
(72) Inventor: ÖHMAN, Peter, S-431 83 Mölndal (SE)
(74) Representative: Miller, Thomas Kerr
(86) International application number: PCT/SE2002/001038
(87) International publication number: WO 2002/096402

(56) References cited:
- WO-A1-00/27401
- WO-A1-99/62870
- WO-A1-99/62872
- RIDDLE MATTHEW: 'Combining sulfonylureas and other oral agents' THE AMERICAN JOURNAL OF MEDICINE vol. 108, no. 6A, 17 April 2000, pages 15S - 22S, XP000925388

## Description

The present invention relates to the use of a combination of certain propanoic acid derivatives which act as peroxisome proliferator activated receptor (PPAR) agonists and a biguanide drug, useful in the treatment of states of insulin resistance, including type 2 diabetes mellitus and associated conditions. Novel pharmaceutical combination compositions are also defined, together with methods of their production.

Traditionally, therapeutic intervention in type 2 diabetes has had a 'glucocentric focus' dominated by the use of insulin secretogogues e.g. the sulfonylureas and the measurement of glycated haemoglobin (HbA1c) or fasting blood sugar level (FPG) as indices of diabetic control. In the USA, patients with type 2 diabetes are usually treated with diet and, when needed, a sulfonylurea compound. However, it is estimated that approximately 30% of patients initially treated with sulfonylurea agents have a poor response and in the remaining 70%, the subsequent failure rate is approximately 4-5% per annum. Other estimates put failure rates higher with few patients responding after 10 years therapy. A treatment-related increase in body weight is also experienced with these agents. Prior to the FDA approval of metformin in 1995, the only therapeutic option for type 2 diabetic patients, in whom sulphonylurea therapy had failed, was insulin.

Despite the introduction of newer agents both the incidence and prevalence of type 2 diabetes continues to increase on a global basis. Approximately 16 million people in the USA have diabetes mellitus, 90-95% of whom have type 2 disease. This represents an enormous healthcare burden; estimated in 1998 to be some $98 billion per annum in direct and indirect healthcare costs. Recently, both the ADA and WHO have revised guidelines for the diagnosis of diabetes and classified diabetes more according to aetiology. The threshold for diagnosis (FPG > 126mg/dl) has been lowered and the term 'type 2' is now used to describe mature onset diabetics. After the ADA implemented these new criteria in 1997, the prevalence of the type 2 disease sector increased by nearly 6 million people in the seven major pharmaceutical markets (France, Germany, Italy, Japan, Spain, UK and USA).

Apart from often mild acute symptoms, type 2 diabetics are also at a considerable risk of developing long term complications of the disease. These include a 4-5 fold higher risk, (compared with non-diabetics), of developing macrovasular disease including CHD and PVD and microvascular complications including retinopathy, nephropathy and neuropathy. In many individuals, overt type 2 diabetes is preceded by a period of reduced insulin sensitivity (insulin resistance), accompanied by a cluster of other cardiovascular risk factors, collectively termed as insulin resistance syndrome (IRS).

It has been estimated that approximately 80% of type 2 diabetics are obese and other co-morbidities of the IRS include: dyslipidemia, hyperinsulinemia, raised arterial blood pressure, uricemia and a reduced fibrinolysis. Given the increased global prevalence and incidence of type 2 diabetes and the very high costs of treating the long term complications of the disease there is tremendous interest in the development of agents that delay or prevent the onset of type 2 diabetes and in those that reduce the risk of cardiovascular complications associated with IRS. These activities have lead to the introduction of the thiazolidinedione (TZD) class of insulin sensitisers that improved the dyslipidemia and thus restored the insulin sensitivity leading to improved glycemic control and lower HbA1c levels.

Although the complex interplay between lipids and carbohydrates as metabolic fuels has been recognised for many decades it is only recently, that researchers and clinicians have begun to focus on the importance of dyslipidemia seen in type 2 diabetes. Much has been made of the relative sensitivities of muscle, liver and adipose tissues to insulin and a case for the primacy of insulin resistance in adipose tissue leading to the IRS has been debated. A typical dyslipidemic atherogenic lipoprotein phenotype (referred to as type B) is seen in IRS including frequently in type 2 diabetics, characterised by a modestly raised LDL-C, a more significant increase in VLDL-TG and reduced HDL. Apparently, changes in the physicochemical properties of VLDL-TG particles result in slower plasma clearance rates and in the generation of small dense LDL particles. The latter permeate the vascular endothelium more readily and are more prone to oxidation and glycation and are considered to play a critical role in atherogenesis in large vessels. Although more difficult to measure, improved free fatty acid (IFFA) flux is increasingly considered to play an important role in the IRS affecting metabolic events in muscle, liver, adipose tissue and pancreas.

The first generation TZDs e.g. troglitazone, pioglitazone, rosiglitazone were in clinical development before the putative mechanism of action was discovered and published in 1995 (PPARγ activation). It is clear from experience with these first generation agents that it is difficult to predict from animal pharmacology the safety and efficacy profile these agents will have in the clinic. Thus, knowledge of the putative mechanism of action of this class coupled with concerns regarding safety, offers the opportunity to identify non-TZD activators of PPAR for the treatment of type 2 diabetes and is the subject of this invention. Furthermore, we recognise that agents with a dual action at both α and γPPAR may have additional benefits in reducing diabetic co-morbidities, particularly raised triglycerides. Such agents may be useful in the treatment of type 2 diabetes, the IRS, dyslipidemia and in reducing risk of cardiovascular disease.

There is considerable unmet need in the treatment of type 2 diabetes, especially for the complications of type 2 diabetes, The University Group Diabetes Program (UGDP) found no benefit from improved glucose control induced by insulin, biguanide or sulfonyurea treatment in preventing diabetic complications over 11 years of follow up in 1,027 patients suffering from type 2 diabetes. The UKPD Study showed effects on microvascular complications. However, data have been interpreted that in obese type 2 diabetics metformin could prevent macrovascular events.

Compounds which have both PPARα and PPARγ agonistic effects are (S)-2-ethoxy-3-[4-(2-{4-methanesulfonyloxyphenyl}ethoxy)phenyl] propanoic acid and 3-{4-[2-(4-*tert-*butoxycarbonylaminophenyl)ethoxy]phenyl}-(S)-2-ethoxy propanoic acid, or a pharmaceutically-acceptable salt thereof and any solvates of either thereof. We believe these compounds to have synergistic properties when used in combination therapy with a "biguanide" drug. By the use of the term biguanide we mean the class of structurally similar drugs referred to in the literature as biguanides which reduce hepatic glucose production and increase peripheral insulin sensitivity, for example metformin, phenformin and buformin.

Specific synergistic effects will be in a more efficient reduction in fasting glucose levels and HbA1c levels in the plasma and an overall positive effect on underlying dyslipidemia. By the use of the term synergistic we mean that more than an additive effect is produced by the drug combination in either efficacy or reduction in side effects. It will be appreciated that as a consequence of synergy lower dosages of one or both active agents may be used when used in combination. It is also expected that a reduction in side effects of each drug may be achieved.

Therefore we present as a feature of the invention a pharmaceutical combination comprising either (S)-2-ethoxy-3-[4-(2-{4-methanesulfonyloxyphenyl}ethoxy)phenyl] propanoic acid or 3-{4-[2-(4-*tert*-butoxycarbonylaminophenyl)ethoxy]phenyl}-(S)-2-ethoxy propanoic acid, or a pharmaceutically-acceptable salt thereof and any solvates thereof and a biguanide drug.

It will be apparent that the combination of the invention may be used alongside other additional existing therapies for the treatment of type 2 diabetes and its associated complications, these include insulin (synthetic insulin analogues, amylin) and oral antihyperglycemics (these are divided into prandial glucose regulators and alpha-glucosidase inhibitors). An example of an alpha-glucosidase inhibitor is acarbose or voglibose or miglitol. An example of a prandial glucose regulator is repaglinide or nateglinide. In addition the combination of the invention may be used in conjunction with another PPAR modulating agent. PPAR modulating agents include but are not limited to thiazolidine-2,4-diones for example troglitazone, ciglitazone, rosiglitazone and pioglitazone. In addition the combination of the invention may be used in conjunction with a sulfonylurea for example: glimepiride, glibenclamide (glyburide), gliclazide, glipizide, gliquidone, chloropropamide, tolbutamide, acetohexamide, glycopyramide, carbutamide, glibonuride, glisoxepid, glybuthiazole, glibuzole, glyhexamide, glymidine, glypinamide, phenbutamide, tolcylamide and tolazamide. Preferably the sulfonylurea is glimepiride or glibenclamide (glyburide). More preferably the sulfonylurea is glimepiride. Therefore the present invention includes administration of a combination of the present invention in conjunction with one, two or more existing therapies described in this paragraph. The doses of the other existing therapies for the treatment of type 2 diabetes and its associated complications will be those known in the art and approved for use by regulatory bodies for example the FDA and may be found in the Orange Book published by the FDA. Alternatively smaller doses may be used as a result of the benefits derived from the combination.

Accordingly, further independent aspects of the present invention include the following:
(1) a pharmaceutical combination comprising (S)-2-ethoxy-3-[4-(2-{4-methanesulfonyloxyphenyl}ethoxy)phenyl] propanoic acid or a pharmaceutically-acceptable salt thereof or a solvate of either thereof and metformin;
(2) a pharmaceutical combination comprising or 3-{4-[2-(4-*tert-*butoxycarbonylaminophenyl)ethoxy]phenyl}-(S)-2-ethoxy propanoic acid or a pharmaceutically-acceptable salt thereof or a solvate of either thereof and metformin.

The pharmaceutical combination' may be achieved by dosing each component drug of the combination to the patient separately in individual dosage forms administered together or sequentially. Alternatively the 'pharmaceutical combination' may be together in the same unit dosage form.

Therefore, as a further aspect of the invention we represent a pharmaceutical composition comprising a pharmaceutical combination as described hereinabove together with a pharmaceutically acceptable carrier and/or diluent.

Independent aspects of the present invention include a pharmaceutical composition comprising (S)-2-ethoxy-3-[4-(2-{4-methanesulfonyloxyphenyl}ethoxy)phenyl] propanoic acid or 3-{4-[2-(4-*tert*-butoxycarbonylaminophenyl)ethoxy]phenyl}-(S)-2-ethoxy propanoic acid, or a pharmaceutically-acceptable salt thereof or a solvate of either thereof and metformin with a pharmaceutically acceptable carrier and/or diluent.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal track, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Suppository formulations may be prepared by mixing the active ingredient with a suitable non-irritating excipient which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedure well known in the art.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on Formulation the reader is referred to Chapter 25.2 in Volume 5 of Comprehensive Medicinal Chemistry (Corwin Hansch; Chairman of Editorial Board), Pergamon Press 1990.

The amount of active ingredients that are combined with one or more excipients to produce a single dosage form will necessarily vary depending upon the host treated and the particular route of administration. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The size of the dose for therapeutic or prophylactic purposes will naturally vary according to the nature and severity of the conditions, the age and sex of the animal or patient and the route of administration, according to well known principles of medicine. For guidance it is suggested that a dose of 0.5 to 25 mg per day , preferably 1 to 10 mg per day, for example 1mg, 2 mg, 3mg, 4mg or 5mg, is used for (S)-2-ethoxy-3-[4-(2-{4-methanesulfonyloxyphenyl }ethoxy)phenyl] propanoic acid or 3-{4-[2-(4-*tert-*butoxycarbonylaminophenyl)ethoxy]phenyl}-(S)-2-ethoxy propanoic acid, or a pharmaceutically-acceptable salt thereof and any solvates of either thereof . For metformin a dose of 250 to 2500 mg per day, preferably 500 to 1700mg per day, is recommended.

Thus in yet a further aspect, the invention provides a use of the compounds for the manufacture of a medicament for treating or preventing diabetes which comprises administering to a patient an effective amount of a pharmaceutical combination as defined above. The invention provides the use of the compounds for the manufacture of a medicament for treating insulin resistance syndrome which comprises administering to a patient in need thereof an effective amount of a pharmaceutical combination as defined above.

A further aspect of the present invention relates to kits of parts comprising:
(i) a vessel containing either (S)-2-ethoxy-3-[4-(2-{4-methanesulfonyloxyphenyl}-ethoxy)phenyl] propanoic acid or 3-{4-[2-(4-*tert*-butoxycarbonylaminophenyl)ethoxy]-phenyl}-(S)-2-ethoxy propanoic acid, or a pharmaceutically-acceptable salt thereof and
(ii) a vessel containing a biguanide drug for example metformin and instructions for the sequential, separate or simultaneous administration of one of the propanoic acids and the biguanide drug to a patient for which such administration is necessary or advantageous.

Another aspect of the invention relates to kits of parts comprising:
(i) a pharmaceutical formulation containing either (S)-2-ethoxy-3-[4-(2-{4-methanesulfonyloxy-phenyl}ethoxy)phenyl] propanoic acid or 3-{4-[2-(4-*tert-*butoxycarbonylaminophenyl)-ethoxy]phenyl}-(S)-2-ethoxy propanoic acid, or a pharmaceutically-acceptable salt thereof in admixture with a pharmaceutically acceptable adjuvant, diluent or carrier; and
(ii) a pharmaceutical formulation containing a biguanide drug for example metformin, in admixture with a pharmaceutically acceptable adjuvant, diluent or carrier; wherein the propanoic acids and the biguanide drug for example metformin are each provided in a form that is suitable for administration in conjunction with the other.

By "administration in conjunction with", we include that respective formulations comprising either propanoic acid and the biguanide drug, for example metformin, are administered, simultaneously, separately or sequentially, over the course of treatment of the relevant condition, which condition may be acute or chronic. Particularly, the term includes that the two formulations are administered (optionally repeatedly) sufficiently closely in time for there to be a beneficial effect for the patient, that is greater, over the course of the treatment of the relevant condition, than if either of the two formulations are administered (optionally repeatedly) alone, in the absence of the other formulation, over the same course of treatment. Preferably the two formulations are administered simultaneously or sequentially, for example in the range of 15 minutes to 12 hours apart, preferably in the range 1 to 8 hours apart.

There is further provided:
(1) a pharmaceutical formulation including either (S)-2-ethoxy-3-[4-(2-{4-methane-sulfonyloxyphenyl}ethoxy)phenyl] propanoic acid or 3-{4-[2-(4-*tert*-butoxycarbonyl-aminophenyl)-ethoxy]phenyl}-(S)-2-ethoxy propanoic acid, or a pharmaceutically-acceptable salt thereof and a biguanide drug, for example metformin, in admixture with a pharmaceutically-acceptable adjuvant, diluent or carrier (which formulation is hereinafter referred to as a "combined preparation"); and
(2) a kit of parts comprising components:
   (a) a pharmaceutical formulation including either (S)-2-ethoxy-3-[4-(2-{4-methane-sulfonyloxyphenyl}ethoxy)phenyl] propanoic acid or 3-{4-[2-(4-*tert*-butoxycarbonyl-aminophenyl)-ethoxy]phenyl}-(S)-2-ethoxy propanoic acid, or a pharmaceutically-acceptable salt thereof in admixture with a pharmaceutically-acceptable adjuvant, diluent or carrier; and
   (b) a pharmaceutical formulation including a biguanide drug, for example metformin, in admixture with a pharmaceutically-acceptable adjuvant, diluent or carrier, which components (a) and (b) are each provided in a form that is suitable for administration in conjunction with the other.

According to a further aspect of the invention, there is provided a method of making a kit of parts as defined above, which method comprises bringing a component (a), as defined above, into association with a component (b), as defined above, thus rendering the two components suitable for administration in conjunction with each other.

By bringing the two components "into association with" each other, we include that components (a) and (b) of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Thus, there is further provided a kit of parts comprising:
(I) one of components (a) and (b) as defined herein; together with
(II) instructions to use that component in conjunction with the other of the two components.

The kits of parts described herein may comprise more than one formulation including either (S)-2-ethoxy-3-[4-(2-{4-methanesulfonyloxyphenyl}ethoxy)phenyl] propanoic acid or 3-{4-[2-(4-*tert*-butoxycarbonylaminophenyl)-ethoxy]phenyl}-(S)-2-ethoxy propanoic acid, or a pharmaceutically-acceptable salt thereof, and/or more than one formulation including an appropriate quantity/dose of a biguanide drug for example metformin, in order to provide for repeat dosing. If more than one formulation (comprising either active compound) is present, such formulations may be the same, or may be different in terms of the dose of either (S)-2-ethoxy-3-[4-(2-{4-methanesulfonyloxyphenyl}ethoxy)phenyl] propanoic acid or 3-{4-[2-(4-*tert*-butoxycarbonylaminophenyl)-ethoxy]phenyl}-(S)-2-ethoxy propanoic acid, or a pharmaceutically-acceptable salt thereof or the biguanide drug for example metformin, chemical composition and/or physical form.

Specifically claimed herein are specific fixed dose combinations where any dose stated for a test compound is combined with any dose stated for the biguanide drug, for example metformin, including the doses stated as limits for the ranges described earlier.

The invention will now be particularly described by way of example. A test compound as used hereafter means either (S)-2-ethoxy-3-[4-(2-{4-methanesulfonyloxyphenyl}ethoxy)-phenyl] propanoic acid or 3-{4-[2-(4-*tert*-butoxycarbonylaminophenyl)-ethoxy]phenyl}-(S)-2-ethoxy propanoic acid.

The advantages of the present invention are demonstrable by administering a) control b) a test compound c) metformin and d) a combination of a test compound and metformin; to genetically obese and diabetic animals, for example Male Wistar rats, fa/fa Zucker rats or ob/ob mice, and measuring plasma glucose levels or lipid levels or another physiological indicator of the insulin resistance syndrome for example glycemic parameters (fasting plasma glucose (FPG), insulin, proinsulin, C-peptide; lipid parameters (triglycerides, total cholesterol, LDL-cholesterol, HDL-cholesterol, total/HDL-cholesterol ratio. LDL/HDL-cholesterol ratio, Apo A1, Apo B, Apo B/Apo A1 ratio, free fatty acids); thrombosis/vascular markers (PAI-1, fibrinogen, urinary albumin/creatinine ratio). A statistical analysis of the results obtained for each compound separately compared to those obtained from the combination may show a synergistic effect.

Alternatively a 26-Week Randomized, Double-Blind, Multicenter, Placebo-Controlled is carried out to Evaluate the Efficacy of a test compound when added to the therapy of patients with Type 2 Diabetes Mellitus poorly Controlled on Metformin alone.

Three doses of test compound will be compared to placebo. Improvements in glycemic control and dyslipidemia are evaluated in patients with type 2 diabetes mellitus who remain poorly controlled (i.e., FPG 126-240 mg/dL) on metformin therapy plus diet/exercise during the placebo run-in period. The study will consist of a screening period (≥2 weeks), a metformin titration period (≤ 4 weeks), a placebo plus metformin run-in period (4 weeks, single-blind, metformin plus placebo plus diet/exercise), a treatment period (26 weeks, double blind), and a follow-up period (3 weeks). All oral antidiabetic medications other than metformin monotherapy are required to be discontinued at the initial screening visit. During the metformin titration period, patients will be titrated to optimal effect, taking into account fasting plasma glucose and safety/tolerability. However, in order to be eligible to continue in the study, patients must be titrated to at least 2.0 g metformin per day. Patients well then enter the placebo run-in period; patients with FPG ≥ 126 mg/dL and ≤ 240 mg/dL during the placebo run-in visits are eligible to enter the treatment period. Patients will be stratified according to prior therapy and gender. Randomization will be performed separately for each of these strata. Patients will be counseled on dietary modification, with reinforcement throughout the treatment period. Any patient with FPG > 270 mg/dL at consecutive visits will be required to be withdrawn from the study. At the end of the treatment period, eligible patients may enter a long-term open-label extension study.

### Inclusion Criteria

Patients may be included in the study if they satisfy the following criteria. Have been diagnosed with type 2 diabetes mellitus (fasting plasma glucose ≥ 126 mg/dl). Patients are eligible if they have been treated with a single or multiple oral agents; however, patients are required to discontinue all antidiabetic medications at the screening visit. Patients are required to have a fasting plasma glucose level of ≥ 126 mg/dL and ≤ 240 mg/dL during the placebo run-in period.
Men or women who are 30 to 80 years of age at the screening visit.
Female patients must be post-menopausal (i.e., ≥ 6 months without a menstrual period), surgically sterile, or using hormonal contraceptives or intrauterine devices. Female patients taking hormonal contraceptives must also be using an additional barrier method of birth control.
Endogenous insulin production as demonstrated by a fasting C-peptide level of ≥ 0.8 ng/mL at the screening and placebo run-in visits.
Fasting triglyceride concentrations at placebo run-in visits must be within 40 percent of each other, using the higher value as the denominator in the calculation (low/high > 0.6) Sign the informed consent to participate.

### Exclusion Criteria

Patients are excluded from the study if they satisfy one or more of the following criteria: Be a diabetic patient previously drug naïve, or treated with chronic insulin therapy or a thiazolidinedione (TZD; glitazone) within 6 months of screening. Patients treated with metformin, a sulfonylurea, a meglitinide, or an alpha glucosidase inhibitor are eligible for enrollment; however, their antidiabetic medications (other than sulfonylurea or metformin) must be discontinued at the screening visit.
Have fasting triglycerides > 600 mg/dL or LDL-C > 250 mg/dL at any visit during the screening and placebo run-in period.
Have uncontrolled hypertension (mean systolic blood pressure ≥ 170 mm Hg or mean diastolic blood pressure ≥ 100 mm Hg). Patients on antihypertensive treatment with a thiazide diuretic, an alpha-adrenergic blocking agent, or a beta-adrenergic blocking agent should be on a constant dose of that medication for at least one month prior to study enrollment and must remain on a constant dose throughout the study, unless medically indicated.
Be treated with fibrates or other lipid lowering agents within 1 month of the screening visit. HMG-CoA reductase inhibitors are allowed, provided that therapy was initiated at least 3 months prior to the screening visit and the dose has remained unchanged for ≥ 3 months prior to the screening visit.
Have a body mass index (BMI) > 40 kg/m² at screening.
Have active arterial disease such as unstable angina, myocardial infarction, transient ischemic attack (TIA), cerebrovascular accident (CVA), coronary artery bypass graft (CABG) surgery, or angioplasty within 3 months of the screening visit.
Have New York Heart Association Class III or IV heart failure.
Have active liver disease or hepatic dysfunction defined by ALT or AST elevations of ≥ 1.5 times the upper limit of normal at any time during the screening or placebo run-in period.
Have experienced previous liver enzyme elevations (> 2.5 times the upper limit of normal) or liver dysfunction while taking troglitazone, ploglitazone, or rosiglitazone.
Have renal impairment defined as a serum creatinine level > 1.4 mg/dL at any time during the screening or placebo run-in period.
Have a hemoglobinopathy or anemia defined as Hgb < I g/dL for males and <10 g/dL for females at any time during the screening or placebo run-in period.
Have a history of malignancy within the last 5 years, excluding successful treatment of basal or squamous cell skin carcinoma.
Pregnancy or lactation.
Have serious or unstable medical or psychological conditions that, in the opinion of the investigator, would compromise the patient's safety or successful participation in the trial. Have any clinically significant abnormality identified on the screening physical examination, laboratory tests, or electrocardiogram, which in the judgment of the investigator would preclude safe completion of the study.
Have a history of alcohol or drug abuse within the last 5 years.
Have an unstable weight as indicated by a > 3 kg change over the 3 months prior to screening.

### Results

The effect of test compound in combination with metformin, on glycemic control, as determined by the mean change from baseline in HbA1c compared to metformin alone.

In addition the mean change from baseline in the metformin + test compound groups with the metformin + placebo groups in the following parameters:
Glycemic parameters (fasting plasma glucose (FPG), insulin, proinsulin, C-peptide; Lipid parameters (triglycerides, total cholesterol, LDL-cholesterol, HDL-cholesterol, total/HDL-cholesterol ratio, LDL/HDL-cholesterol ratio, Apo A1, Apo B, Apo B/Apo A I ratio, free fatty acids);
Thrombosis/vascular markers (PAI-1, fibrinogen, urinary albumin/creatinine ratio).

In addition, the following will be evaluated:
Responder analyses for HbA1c (proportion of patients with reductions from baseline of at least 0.7% and 1%); FPG (proportion of patients with reductions from baseline of at least 30 mg/dL), and TG (proportion of patients with reductions from baseline of at least 20% and 40%);
Proportion of patients reaching target goals for HbA1c (≤ 8% and ≤ 7%); FPG (≤ 126 mg/dL); and TG (≤ 200 and ≤ 150 mg/dL);
HOMA: percentage change from baseline in insulin sensitivity and β-cell function.

Clinical safety and tolerability, as assessed by changes in physical examinations, vital signs, body weight, clinical laboratory tests (including lactic acid), adverse experiences, and electrocardiograms

Patients will receive metformin as background therapy in an open label fashion. For each background therapy, three doses of test compound will be used: two top doses and one starting dose, given as a single daily dose for a duration of 26 weeks. If any safety concerns are raised with the highest dose during the 6-month trials, then the second top dose will be available for continued development. In addition, a placebo will be used as a comparator.

Analysis of the results is expected to demonstrate one or more of the following:
significant improvement in glycemic control compared to baseline and placebo for a combination of the test compound with metformin;
improvement in lipid profile compared to baseline and compared to placebo for a combination of the test compound with metformin;
most patients are "responders" for glycemic and triglyceride control in combination with metformin;
most patients will achieve target goals for glycemic and lipid control in combination with metformin;
for the test compound in combination with metformin, effective glycemic and lipid control regardless of baseline BMI, age, gender, race, or severity of disease; no clinically relevant increases in weight.

It is also expected that statistical analysis of the above results will demonstrate that the combination of the test compound and insulin has a synergistic effect on one or more of the physiological responses measured.

## Claims

1. A pharmaceutical combination comprising either (S)-2-ethoxy-3-[4-(2-{4-methanesulfonyloxyphenyl}ethoxy)phenyl] propanoic acid or 3-{4-[2-(4-*tert-*butoxycarbonylaminophenyl)ethoxy]phenyl}-(S)-2-ethoxy propanoic acid, or a pharmaceutically-acceptable salt thereof and any solvates of either thereof and a biguanide drug.

2. A pharmaceutical combination according to claim 1 comprising (S)-2-ethoxy-3-[4-(2-{4-methanesulfonyloxyphenyl}ethoxy)phenyl] propanoic acid or a pharmaceutically-acceptable salt thereof or a solvate of either thereof and metformin.

3. A pharmaceutical combination according to claim 1 comprising 3-{4-[2-(4-*tert-*butoxycarbonylaminophenyl)ethoxy]phenyl}-(S)-2-ethoxy propanoic acid or a pharmaceutically-acceptable salt thereof or a solvate of either thereof and metformin.

4. A pharmaceutical composition comprising (S)-2-ethoxy-3-[4-(2-{4-methanesulfonyloxyphenyl}ethoxy)phenyl] propanoic acid or 3-{4-[2-(4-*tert-*butoxycarbonylaminophenyl)ethoxy]phenyl}-(S)-2-ethoxy propanoic acid, or a pharmaceutically-acceptable salt thereof or a solvate of either thereof and metformin with a pharmaceutically acceptable carrier and/or diluent.

5. The use of either (S)-2-ethoxy-3-[4-(2-{4-methanesulfonyloxyphenyl}ethoxy)phenyl] propanoic acid or 3-{4-[2-(4-*tert*-butoxycarbonylaminophenyl)ethoxy]phenyl}-(S)-2-ethoxy propanoic acid, or a pharmaceutically-acceptable salt thereof and any solvates of either thereof and a biguanide drug in the manufacture of a medicament for treating or preventing diabetes.

6. The use of either (S)-2-ethoxy-3-[4-(2-{4-methanesulfonyloxyphenyl}ethoxy)phenyl] propanoic acid or 3-{4-[2-(4-*tert*-butoxycarbonylaminophenyl)ethoxy]phenyl}-(S)-2-ethoxy propanoic acid, or a pharmaceutically-acceptable salt thereof and any solvates of either thereof and a biguanide drug in the manufacture of a medicament for treating insulin resistance syndrome.

7. A kit of parts comprising:
(i) a vessel containing either (S)-2-ethoxy-3-[4-(2-{4-methanesulfonyloxyphenyl}-ethoxy)phenyl] propanoic acid or 3-{4-[2-(4-*tert*-butoxycarbonylaminophenyl)ethoxy]-phenyl}-(S)-2-ethoxy propanoic acid, or a pharmaceutically-acceptable salt thereof and
(ii) a vessel containing a biguanide drug
and instructions for the sequential, separate or simultaneous administration of one of the propanoic acids and the biguanide drug to a patient for which such administration is necessary or advantageous.

8. A kit of parts comprising:
(i) a pharmaceutical formulation containing either (S)-2-ethoxy-3-[4-(2-{4-methanesulfonyloxy-phenyl}ethoxy)phenyl] propanoic acid or 3-{4-[2-(4-*tert-*butoxycarbonylaminophenyl)-ethoxy]phenyl}-(S)-2-ethoxy propanoic acid, or a pharmaceutically-acceptable salt thereof in admixture with a pharmaceutically acceptable adjuvant, diluent or carrier; and
(ii) a pharmaceutical formulation containing a biguanide drug in admixture with a pharmaceutically acceptable adjuvant, diluent or carrier;
wherein the propanoic acids and the biguanide drug are each provided in a form that is suitable for administration in conjunction with the other.

9. A combination product comprising:
(1) a pharmaceutical formulation including either (S)-2-ethoxy-3-[4-(2-{4-methane-sulfonyloxyphenyl}ethoxy)phenyl] propanoic acid or 3-{4-[2-(4-*tert*-butoxycarbonyl-aminophenyl)-ethoxy]phenyl}-(S)-2-ethoxy propanoic acid, or a pharmaceutically-acceptable salt thereof and a biguanide drug in admixture with a pharmaceutically-acceptable adjuvant, diluent or carrier; and
(2) a kit of parts comprising components:
(a) a pharmaceutical formulation including either (S)-2-ethoxy-3-[4-(2-{4-methane-sulfonyloxyphenyl}ethoxy)phenyl] propanoic acid or 3-{4-[2-(4-*tert*-butoxycarbonyl-aminophenyl)-ethoxy]phenyl}-(S)-2-ethoxy propanoic acid, or a pharmaceutically-acceptable salt thereof in admixture with a pharmaceutically-acceptable adjuvant, diluent or carrier; and
(b) a pharmaceutical formulation including a biguanide drug in admixture with a pharmaceutically-acceptable adjuvant, diluent or carrier,
which components (a) and (b) are each provided in a form that is suitable for administration in conjunction with the other.

10. A method of making a combination product as defined in claim 9, which method comprises bringing a component (a), as defined in claim 9, into association with a component (b), as defined in claim 9, thus rendering the two components suitable for administration in conjunction with each other.

11. A method according to claim 10 wherein (a) and (b) of the kit of parts may be:
(i) provided as separate formulations independently of one another, which are subsequently brought together for use in conjunction with each other in combination therapy; or
(ii) packaged and presented together as separate components of a "combination pack" for use in conjunction with each other in combination therapy.

12. A kit of parts comprising:
(I) one of components (a) and (b) as defined in claim 9; together with
(II) instructions to use that component in conjunction with the other of the two components.

13. A kit of parts according to any one of claims 7, 8 or 12 which comprises more than one formulation including either (S)-2-ethoxy-3-[4-(2-{4-methanesulfonyloxyphenyl}ethoxy)phenyl] propanoic acid or 3-{4-[2-(4-*tert*-butoxycarbonylaminophenyl)-ethoxy]phenyl}-(S)-2-ethoxy propanoic acid, or a pharmaceutically-acceptable salt thereof, and/or more than one formulation including an appropriate quantity/dose of a biguanide drug in order to provide for repeat dosing.

14. A pharmaceutical combination according to any one of claims 1, 2 or 3 or a pharmaceutical composition according to claim 4 or a use according to either one of claims 5 or 6, or a method according to either claim 10 or claim 11 or a kit of parts according to any one of claims 7, 8, 12 or 13 or a combination product according to claim 9 which further comprises one or more additional existing therapies for the treatment of type 2 diabetes and its associated complications.

15. A pharmaceutical combination according to claim 1 or a use according to either one of claims 5 or 6, or a method according to either claim 10 or claim 11 or a kit of parts according to any one of claims 7, 8, 12 or 13 or a combination product according to claim 9 wherein the biguanide drug is metformin.

16. A pharmaceutical composition according to claim 4 or a use according to either one one of claims 5 or 6 or a method according to either claim 10 or claim 11 or a kit of parts according to any one of claims 7, 8, 12 or 13 or a combination product according to claim 9 wherein the biguanide drug is metformin and the propanoic acid is (S)-2-ethoxy-3-[4-(2-{4-methanesulfonyloxyphenyl}ethoxy)phenyl] propanoic acid.

## Patentansprüche

1. Pharmazeutische Kombination, die entweder (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze und beliebige Solvate von einer dieser Substanzen sowie einen Biguanid-Arzneistoff umfaßt.

2. Pharmazeutische Zusammensetzung nach Anspruch 1, die (S)-2-Ethoxy-3-[4-(2-{4-methansulfonyloxyphenyl}ethoxy)phenyl]propansäure oder eines ihrer pharmazeutisch annehmbaren Salze oder ein Solvat von einer dieser Substanzen sowie Metformin umfaßt.

3. Pharmazeutische Zusammensetzung nach Anspruch 1, die 3-{4-[2-(4-*tert*.-Butoxycarbonylaminophenyl)ethoxy]phenyl}-(S)-2-ethoxypropansäure oder eines ihrer pharmazeutisch annehmbaren Salze oder ein Solvat von einer dieser Substanzen sowie Metformin umfaßt.

4. Pharmazeutische Kombination, die entweder (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze oder ein Solvat von einer dieser Substanzen sowie Metformin gemeinsam mit einem pharmazeutisch annehmbaren Träger und/oder Verbindungsmittel umfaßt.

5. Verwendung von (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze und beliebige Solvate von einer dieser Substanzen sowie einem Biguanid-Arzneistoff bei der Herstellung eines Arzneimittels für die Behandlung von bzw. Vorbeugung gegen Diabetes.

6. Verwendung von (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze und beliebige Solvate von einer dieser Substanzen sowie einem Biguanid-Arzneistoff bei der Herstellung eines Arzneimittels für die Behandlung des Insulinresistenzsyndroms.

7. Aus Teilen bestehendes Kit, das folgendes umfaßt:
(i) ein Behältnis, das (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze enthält, sowie
(ii) ein Behältnis, das einen Biguanid-Arzneistoff enthält,
sowie Anweisungen für die aufeinanderfolgende, getrennte oder gleichzeitige Verabreichung von einer der Propansäuren und dem Biguanid-Arzneistoff an einen Patienten, für den solch eine Verabreichung erforderlich oder vorteilhaft ist.

8. Aus Teilen bestehendes Kit, das folgendes umfaßt:
(i) eine pharmazeutische Formulierung, die (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze in Abmischung mit einem pharmazeutisch annehmbaren Hilfsstoff, Verdünnungsmittel oder Träger enthält; sowie
(ii) eine pharmazeutische Formulierung, die einen Biguanid-Arzneistoff in Abmischung mit einem pharmazeutisch annehmbaren Hilfsmittel, Verbindungsmittel oder Träger enthält;
wobei die Propansäure und der Biguanid-Arzneistoff jeweils in einer Form bereitgestellt wird, die sich für die Verabreichung gemeinsam mit der anderen Substanz eignet.

9. Kombinationsprodukt, das folgendes enthält:
(1) eine pharmazeutische Formulierung, die (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze und einen Biguanid-Arzneistoff in Abmischung mit einem pharmazeutisch annehmbaren Hilfsstoff, Verdünnungsmittel oder Träger beinhaltet; sowie
(2) ein aus Teilen bestehendes Kit, das die folgenden Komponenten enthält:
(a) eine pharmazeutische Formulierung, die (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze in Abmischung mit einem pharmazeutisch annehmbaren Hilfsstoff, Verdünnungsmittel oder Träger beinhaltet; sowie
(b) eine pharmazeutische Formulierung, die einen Biguanid-Arzneistoff in Abmischung mit einem pharmazeutisch annehmbaren Hilfsmittel, Verbindungsmittel oder Träger umfaßt;
wobei die Komponenten (a) und (b) jeweils in einer Form bereitgestellt werden, in einer Form bereitgestellt wird, die sich für die Verabreichung gemeinsam mit der anderen Substanz eignet.

10. Verfahren zur Herstellung eines in Anspruch 9 definierten Kombinationsprodukts, das umfaßt, daß man eine in Anspruch 9 definierte Komponente (a) mit einer in Anspruch 9 definierten Komponente (b) zusammenbringt und so die beiden Komponenten für eine Verabreichung zusammen mit den jeweils anderen Komponente geeignet macht.

11. Verfahren nach Anspruch 10, wobei (a) und (b) des aus Teilen bestehenden Kits
(i) unabhängig voneinander als getrennte Formulierungen bereitgestellt werden, anschließend für die Verwendung gemeinsam mit der jeweils anderen Komponente in der Kombinationstherapie zusammengegeben werden; oder
(ii) gemeinsam als getrennte Komponenten eines "Kombi-Packs" für die Verwendung gemeinsam mit der jeweils anderen Komponente der Kombinationstherapie verpackt und dargereicht werden.

12. Aus Teilen bestehendes Kit, das folgendes umfaßt:
(I) eine der in Anspruch 9 definierten Komponenten (a) und (b) gemeinsam mit
(II) Anweisungen, diese Komponente zusammen mit der anderen der beiden Komponenten zu verwenden.

13. Aus Teilen bestehendes Kit nach einem der Ansprüche 7, 8 oder 12, das mehr als eine Formulierung, die entweder (S)-2-Ethoxy-3-[4-(2-{4-methansulfonyloxyphenyl}ethoxy)phenyl]propansäure oder 3-{4-[2-(4-*tert*.-Butoxycarbonylaminophenyl)ethoxy]phenyl}-(S)-2-ethoxypropansäure oder eines ihrer pharmazeutisch annehmbaren Salze beinhaltet, und/oder mehr als eine Formulierung, die eine geeignete Menge/Dosis eines Biguanid-Arzneistoffs enthält, umfaßt, um eine Wiederholungsdosierung zu ermöglichen.

14. Pharmazeutische Kombination nach einem der Ansprüche 1, 2 oder 3 bzw. pharmazeutische Zusammensetzung nach Anspruch 4 bzw. Verwendung nach einem der Ansprüche 5, 6, 10 oder 11 bzw. aus Teilen bestehendes Kit nach einem der Ansprüche 7, 8, 12 oder 13 bzw. Kombinationsprodukt nach Anspruch 9, die/das weiterhin eine oder mehrere zusätzliche bereits existierende Therapien für die Behandlung der Typ-2-Diabetes und den damit einhergehenden Komplikationen umfaßt.

15. Pharmazeutische Kombination nach Anspruch 1 bzw. Verfahren nach einem der Ansprüche 5, 6, 10 oder 11 bzw. aus Teilen bestehendes Kit nach einem der Ansprüche 7, 8, 12 oder 13 bzw. Kombinationsprodukt nach Anspruch 9, wobei es sich bei dem Biguanid-Arzneistoff um Metformin handelt.

16. Pharmazeutische Zusammensetzung nach Anspruch 4 oder Verwendung nach Anspruch 5 oder 6 oder Verfahren nach Anspruch 10 oder Anspruch 11 oder aus Teilen bestehendes Kit nach einem der Ansprüche 7, 8, 12 oder 13 oder Kombinationsprodukt nach Anspruch 9, wobei es sich bei dem Biguanid-Arzneistoff um Metformin und bei der Propansäure um (S)-2-Ethoxy-3-[4-(2-{4-methansulfonyloxyphenyl}ethoxy)phenyl]propansäure handelt.

## Revendications

1. Combinaison pharmaceutique comprenant soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou un sel pharmaceutiquement acceptable de ceux-ci et n'importe quel solvate de l'un ou de l'autre et un médicament renfermant un biguanide.

2. Combinaison pharmaceutique selon la revendication 1, comprenant de l'acide (S)-2-éthoxy-3-[4-(2-{4-méthanesulfonyloxyphényl}éthoxy)phényl]propanoïque ou un sel pharmaceutiquement acceptable de celui-ci ou un solvate de l'un ou de l'autre et de la metformine.

3. Combinaison pharmaceutique selon la revendication 1, comprenant de l'acide 3-{4-[2-(4-*tert-*butoxycarbonylaminophényl)éthoxy]phényl}-(S)-2-éthoxy propanoïque ou un sel pharmaceutiquement acceptable de celui-ci ou un solvate de l'un ou de l'autre et de la metformine.

4. Composition pharmaceutique comprenant de l'acide (S)-2-éthoxy-3-[4-(2-{4-méthanesulfonyloxyphényl}éthoxy)phényl]propanoïque ou de l'acide 3-{4-[2-(4-*tert*-butoxycarbonylaminophényl)éthoxy]phényl}-(S)-2-éthoxy propanoïque, ou un sel pharmaceutiquement acceptable de ceux-ci ou un solvate de l'un ou de l'autre et de la metformine avec un véhicule et/ou un diluant pharmaceutiquement acceptable(s).

5. Utilisation soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou d'un sel pharmaceutiquement acceptable de ceux-ci et de n'importe quel solvate de l'un ou de l'autre et d'un médicament renfermant un biguanide pour fabriquer un médicament destiné à traiter ou à prévenir le diabète.

6. Utilisation soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou d'un sel pharmaceutiquement acceptable de ceux-ci et de n'importe quel solvate de l'un ou de l'autre et d'un médicament renfermant un biguanide pour fabriquer un médicament destiné à traiter le syndrome d'insulino-résistance.

7. Trousse de pièces comprenant :
(i) un récipient contenant soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou un sel pharmaceutiquement acceptable de ceux-ci et
(ii) un récipient contenant un médicament renfermant un biguanide
et des instructions pour l'administration séquentielle, distincte ou simultanée d'un des acides propanoïques et du médicament renfermant un biguanide chez un patient pour lequel une telle administration est nécessaire ou bénéfique.

8. Trousse de pièces comprenant :
(i) une formulation pharmaceutique contenant soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou un sel pharmaceutiquement acceptable de ceux-ci mélangé à un adjuvant, un diluant ou un véhicule pharmaceutiquement acceptable ; et
(ii) une formulation pharmaceutique contenant un médicament renfermant un biguanide mélangé à un adjuvant, un diluant ou un véhicule pharmaceutiquement acceptable ;
dans laquelle les acides propanoïques et le médicament renfermant un biguanide sont chacun fournis sous une forme qui est adaptée pour être administrée conjointement avec l'autre composant.

9. Produit combiné comprenant :
(1) une formulation pharmaceutique contenant soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou un sel pharmaceutiquement acceptable de ceux-ci et un médicament renfermant un biguanide mélangé à un adjuvant, un diluant ou un véhicule pharmaceutiquement acceptable ; et
(2) une trousse de pièces comprenant les composants suivants :
(a) une formulation pharmaceutique contenant soit de l'acide (S)-2-éthoxy-3-[4-(2-{4-méthanesulfonyloxyphényl}éthoxy)phényl]propanoïque, soit de l'acide 3-{4-[2-(4-*tert*-butoxycarbonylaminophényl)éthoxy]phényl}-(S)-2-éthoxy propanoïque, ou un sel pharmaceutiquement acceptable de ceux-ci mélangé à un adjuvant, un diluant ou un véhicule pharmaceutiquement acceptable ; et
(b) une formulation pharmaceutique comprenant un médicament renfermant un biguanide mélangé à un adjuvant, un diluant ou un véhicule pharmaceutiquement acceptable,
lesquels composants (a) et (b) sont fournis chacun sous une forme qui est adaptée pour être administrée conjointement avec l'autre composant.

10. Procédé de fabrication d'un produit combiné selon la revendication 9, lequel procédé comprend le fait d'associer un composant (a), selon la revendication 9, à un composant (b), selon la revendication 9, rendant ainsi les deux composants adaptés pour être administrés conjointement l'un avec l'autre.

11. Procédé selon la revendication 10, dans lequel (a) et (b) de la trousse de pièces peuvent être :
(i) fournis sous forme de formulations distinctes indépendamment l'un de l'autre, qui sont ensuite réunis pour être utilisés conjointement l'un avec l'autre en thérapie combinée ; ou
(ii) conditionnés et présentés ensemble en tant que composants distincts d'un « lot combiné » pour une utilisation l'un conjointement avec l'autre en thérapie combinée.

12. Trousse de pièces comprenant :
(I) l'un des composants (a) et (b) selon la revendication 9 ; conjointement avec
(II) un mode d'emploi de ce composant conjointement avec l'autre des deux composants.

13. Trousse de pièces selon l'une quelconque des revendications 7, 8 ou 12 qui comprend plus d'une formulation comprenant soit de l'acide (S)-2-éthoxy-3-[4-(2-{4-méthanesulfonyloxyphényl}éthoxy)phényl]-propanoïque, soit de l'acide 3-{4-[2-(4-*tert-*butoxycarbonylaminophényl)éthoxy]phényl}-(S)-2-éthoxy propanoïque, ou un sel pharmaceutiquement acceptable de ceux-ci et/ou plus d'une formulation comprenant une quantité/dose appropriée d'un médicament renfermant un biguanide afin de fournir un dosage répété.

14. Combinaison pharmaceutique selon l'une quelconque des revendications 1, 2 ou 3 ou composition pharmaceutique selon la revendication 4 ou utilisation selon l'une ou l'autre des revendications 5 et 6, ou procédé selon la revendication 10 ou 11 ou trousse de pièces selon l'une quelconque des revendications 7, 8, 12 ou 13 ou produit combiné selon la revendication 9, qui comprend en outre une ou plusieurs thérapie(s) existante(s) supplémentaire(s) pour traiter le diabète de type 2 et ses complications associées.

15. Combinaison pharmaceutique selon la revendication 1 ou utilisation selon l'une ou l'autre des revendications 5 et 6, ou procédé selon la revendication 10 ou 11 ou trousse de pièces selon l'une quelconque des revendications 7, 8, 12 ou 13 ou produit combiné selon la revendication 9, dans laquelle/lequel le médicament renfermant un biguanide est la metformine.

16. Composition pharmaceutique selon la revendication 4 ou utilisation selon l'une ou l'autre des revendications 5 et 6 ou procédé selon la revendication 10 ou 11 ou trousse de pièces selon l'une quelconque des revendications 7, 8, 12 ou 13 ou produit combiné selon la revendication 9, dans laquelle/lequel le médicament renfermant un biguanide est la metformine et l'acide propanoïque est l'acide (S)-2-éthoxy-3-[4-(2-{4-méthanesulfonyloxyphényl}éthoxy)phényl]propanoïque.
